(19) Europäisches Patentamt
European Patent Office
Office européen des brevets

(11) Veröffentlichungsnummer: **0 429 907 B1**

(12) **EUROPÄISCHE PATENTSCHRIFT**

(45) Veröffentlichungstag der Patentschrift: **01.06.94**

(51) Int. Cl.[5]: **G01N 33/52**, G01N 33/53, G01N 33/533, G01N 33/58, G01N 33/542, G01N 33/553, G01N 33/552, G01N 33/545

(21) Anmeldenummer: **90121347.0**

(22) Anmeldetag: **08.11.90**

(54) **Optischer Biosensor.**

(30) Priorität: **21.11.89 DE 3938598**
**28.04.90 DE 4013713**

(43) Veröffentlichungstag der Anmeldung:
**05.06.91 Patentblatt 91/23**

(45) Bekanntmachung des Hinweises auf die Patenterteilung:
**01.06.94 Patentblatt 94/22**

(84) Benannte Vertragsstaaten:
**AT BE CH DE DK FR GB IT LI NL SE**

(56) Entgegenhaltungen:
**EP-A- 0 150 905**
**EP-A- 0 174 744**

**WPIL, FILE SUPPLIER, Derwent Publications Ltd., London (GB); Acc. Nr. 88-033622&NUM;**

**CHEMICAL ABSTRACTS, Band 112, Nr. 4, 22 Januar 1990, Columbus, OH (US); B.B.P. SCHAFFAR et al., Seite 631, Nr. 29895n&NUM;**

(73) Patentinhaber: **BAYER AG**

**D-51368 Leverkusen(DE)**

(72) Erfinder: **Hugl, Herbert, Dr.**
**Gemarkenweg 9**
**W-5060 Bergisch Gladbach 2(DE)**
Erfinder: **Kuckert, Eberhard, Dr.**
**c/o Molecular Diagnostics Inc.,**
**400 Morgan Lane**
**West Haven, CT 06516(US)**
Erfinder: **Möbius, Dietmar, Dr.**
**Kampenweg 4**
**W-3401 Waake(DE)**
Erfinder: **Ohst, Holger, Dr.**
**Im Wiesengrund 10**
**W-5068 Odenthal(DE)**
Erfinder: **Rolf, Meinhard, Dr.**
**c/o Mobay Corporation**
**Charleston, SC 29411(US)**
Erfinder: **Rosenkranz, Hans Jürgen, Dr.**
**Heinrich-Kauert-Weg 9**
**W-4150 Krefeld 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 429 907 B1**

Rank Xerox (UK) Business Services
(3.10/3.09/3.3.3)

**CHEMICAL ABSTRACTS, Band 110, Nr. 6, 06 Februar 1989, Columbus, OH (US); M. AIZAWA et al., Seite 787, Nr. 50443g&NUM;**

**NATURE, Band 320, 13 März 1986, London (GB); T.H. WATTS et al., Seiten 179-181&NUM;**

**ANNUAL REVIEWS OF BIOCHEMISTRY, Band 47, 1978, Palo Alto, CA (US); L. STRYER, Seiten 819-846&NUM;**

Erfinder: **Schopper, Heinrich Christian, Dr.**
**Glindholzstrasse 95a**
**W-4150 Krefeld 1(DE)**
Erfinder: **Siegmund, Hans-Ulrich, Dr.**
**Roonstrasse 100**
**W-4150 Krefeld 1(DE)**
Erfinder: **Sommer, Klaus, Dr.**
**Morgengraben 3**
**W-5000 Köln 80(DE)**
Erfinder: **Wehrmann, Rolf, Dr.**
**Scheiblerstrasse 81**
**W-4150 Krefeld 1(DE)**

## Beschreibung

Die vorliegende Erfindung betrifft einen optischen Biosensor mit einem neuartigen Aufbau für eine Detektionsmethode für Moleküle, die mit einem Fluoreszenzfarbstoff markiert sind, zur Erkennung gelöster Substanzen oder gelöster Analyte, die sich beispielsweise wie Antigen und Antikörper verhalten. Es handelt sich hierbei um einen Festphasensensor mit Fluoreszenzfarbstoff, der einen Energietransferprozeß auf ein zu detektierendes, mit einem zweiten Fluoreszenzfarbstoff markiertes Molekül erlaubt.

Es gibt verschiedene Methoden, Analyten, wie Hormone, Enzyme, andere Proteine, Kohlenhydrate, Nucleinsäuren, pharmakologische Wirkstoffe, Toxine und andere, in flüssigen Proben biologischen Ursprungs nachzuweisen. Unter den bekannten Methoden ragen speziell Immunoassays als eine empfindliche Nachweismethode zur Bestimmung sehr kleiner Mengen organischer Substanzen heraus. Immunoassay-Methoden beruhen allgemein auf der Fähigkeit eines Rezeptormoleküls, beispielsweise eines Antikörpers, die Struktur und molekulare Organisation eines Ligandenmoleküls, sei sie durch unpolare und/oder polare Wechselwirkungen definiert, spezifisch zu erkennen und dieses Molekül auf derartige Weise ganz spezifisch zu binden.

Immunoassays werden mit verschiedenen Methoden durchgeführt. Dazu zählen der Einsatz verschiedener Markierungstechniken, meist radioaktiver, enzymgekoppelter und auch fluoreszierender Natur (Methods in Enzymology, 74 (1981), 28-60).

Einige dieser bekannten Immunoassay-Methoden beinhalten die Verwendung von Fluoreszenzfarbstoff-Molekülen $F_1$, die in der Lage sind, Licht einer Wellenlänge $\lambda_1$ zu absorbieren und Licht einer zweiten, größeren Wellenlänge $\lambda_2$ zu emittieren. In Gegenwart eines anderen Fluoreszenzfarbstoff-Moleküls $F_2$ erfolgt unter bestimmten Bedingungen nach der Anregung von $F_1$ durch Licht der Wellenlänge $\lambda_1$ eine strahlungslose Energieübertragung auf $F_2$, der dann seinerseits Licht einer dritten, noch größeren Wellenlänge $\lambda_3$ emittiert.

Dieses Prinzip des Energietransfers ist von Förster in der Theorie beschrieben worden und ist Anregung für vielerlei mögliche Anwendungen gewesen (Annual Reviews in Biochemistry 47 (1978), 819-846). Eine wichtige Eigenschaft dieses Energietransfers ist dabei seine Abstandsabhängigkeit. Die Wirksamkeit der Energieübertragung nach Förster wird durch den kritischen Radius $R_o$, nämlich den Abstand zwischen Donor und Akzeptor, beschrieben, bei dem der intermolekulare Energietransfer gleich wahrscheinlich wie die Summe aller anderen Desaktivierungsprozesse des Donors ist. Dieser Abstand beträgt etwa 50-100 Å.

Es sind bereits Immunoassays beschrieben worden, die auf der Ausnützung des abstandsabhängigen Energietransfers beruhen. So wird in EP 150 905 ein in homogener Lösung arbeitender Immunoassay beschrieben, in dem Analyt bzw. Antigen mit einem Fluoreszenzfarbstoff $F_1$ markiert und der dazu spezifisch bindende Antikörper mit einem Fluoreszenzfarbstoff $F_2$ versehen wurde. Zum Nachweis der spezifischen Bindung und damit als analytische Methode wird die Tatsache genützt, daß beim Einstrahlen von Licht der Wellenlänge $\lambda_1$ eine Emission der Wellenlänge $\lambda_3$ nur dann beobachtet werden kann, wenn Analyt und Antikörper in ausreichender Konzentration in für eine Energieübertragung nach Förster genügend kleinem Abstand vorliegen. Dies ist nur dann der Fall, wenn Analyt und Antikörper eine spezifische Bindung eingegangen sind.

In einem anderen Beispiel wird einer der beiden markierten Bindungspartner an einer Festkörperoberfläche angebracht und der entsprechend spezifisch bindende Partner aus einer homogenen Lösung gebunden. Wieder erfolgt der Nachweis der spezifischen Bindung, wie oben bereits erläutert, durch einen entsprechenden Energietransfer mittels der Evanescent-wave-Technologie (Nature 320 (1986), 179-181).

Sowohl der hier erwähnte Energietransfer in homogener Lösung als auch der beschriebene Festphasen-Immunoassay mit Energietransfer haben prinzipiell den Nachteil, daß die spezifisch miteinander bindenden Moleküle jeweils mit einem der beiden notwendigen Fluoreszenzfarbstoffe $F_1$ bzw. $F_2$ markiert werden müssen und gemäß Nature 320 (1986), 179-181 ein Verhältnis $F_1$:$F_2$ von maximal 2:1 gestatten.

Es sind bereits Methoden beschrieben worden, mit denen die durch das Verhältnis der beiden Fluoreszenzfarbstoffe $F_1$ und $F_2$ limitierte Empfindlichkeit des fluoreszenzspektroskopischen Nachweises zu verbessern ist. So wird in EP 174 744 vorgeschlagen, mehrere organische Farbstoffmoleküle gleichzeitig an ein "lichtsammelndes" Protein kovalent zu binden, d.h. es erfolgt ein Energietransfer mehrerer organischer Farbstoffmoleküle an nur ein Akzeptormolekül, nämlich in EP 174 744 ein Phycobiliprotein (Allophycocyanin). Dieses Molekülsystem wiederum wird dann als ein "Marker" für andere biologische Moleküle vorgeschlagen. Die Methode wird begrenzt durch das Farbstoff:Protein-Kupplungsverhältnis.

Weiterhin ist ein Nachteil der aufgeführten Systeme dadurch gegeben, daß komplementäre Systeme jeweils spezifisch markiert werden müssen und damit nicht vielseitig verwendet werden können. Ein weiterer Nachteil dieser in heterogener Phase aufgebauten Systeme ist die speziell verwendete Evanes-

cent-wave-Technik. Zudem ist die Fixierung der spezifisch bindenden Moleküle an die Festkörperoberfläche über ein System aus Kupplungskomponente/Antikörper/Antigen/Antikörper präparativ sehr aufwendig. Ein weiterer prinzipieller Nachteil dieser Festphasen-Technologie bei Immunoassays ist die reproduzierbare Herstellung von Beschichtungen der Testmatrix mit den Reaktanden der Immunreaktion. Für analytische Methoden ist jedoch neben Empfindlichkeit und Selektivität bezüglich einer Zielsubstanz die Reproduzierbarkeit der Detektionsmethode ein wesentliches Qualitätsmerkmal.

Die vorliegende Erfindung betrifft einen neuartig aufgebauten Sensor für eine Detektionsmethode von mit Fluoreszenzfarbstoff markierten Molekülen zur Erkennung dieser gelösten Substanzen oder Analyte durch Energietransfer mit einer einfachen Fluoreszenztechnik und gesteigerter Empfindlichkeit in der Detektion sowie vielseitiger Anwendung bei unterschiedlichen Aufgaben und reproduzierbarer Herstellungsmöglichkeit für an Festkörperoberflächen gebundene Schichten. Neben dem deutlichen Gewinn an Sensitivität werden alle oben aufgeführten Nachteile gleichzeitig vermieden.

Die Erfindung betrifft einen optischen Biosensor auf der Basis des Fluoreszenz-Energietransfers, bestehend aus

a) einem festen Träger,

b) einer auf a) angebrachten ein- oder mehrlagigen Langmuir-Blodgett(LB)-Schicht,

c) mindestens einem Fluoreszenzfarbstoff $F_1$, der in mindestens einer der oberen 4 Lagen der LB-Schicht angeordnet ist,

d) einem zur spezifischen Wechselwirkung befähigten Rezeptor-Molekül, das in oder an der obersten Lage der LB-Schicht gebunden oder angeordnet ist, und

e) einem mobilen Fluoreszenzfarbstoff $F_2$, dessen Anregungsbande für einen Energietransfer ausreichend mit der Emissionsbande von $F_1$ überlappt und der

e1) kovalent an einen Liganden gebunden ist, der an den Rezeptor bindungsfähig ist oder der

e2) kovalent an einen anderen Rezeptor gebunden ist, der an den Komplex aus erstem Rezeptor und Ligand bindungsfähig ist,

wobei der Ligand bzw. der Ligand und der zweite Rezeptor zunächst nicht an die LB-Schicht gebunden sind.

Optische Biosensoren nach den Ansprüchen 2-10 sind ebenfalls gegenstand der Anmeldung.

Als Träger kommen alle dem Fachmann bekannten für die LB-Technik geeigneten Träger in Frage, wie Glas, Quarzglas, weitere Gläser, wie Li-niobat, Zinkselenid, Porzellan, Halbleitermaterialien, wie Germanium, GaAs oder Silizium sowie Metalle.

Weiterhin sind geeignet: Kunststoffe, wie Polymethylmethacrylat, Polycarbonat, Polystyrol und andere sowie metallisierte Kunststoffe. Die festen Trägermaterialien können vor der Beschichtung auch oberflächenmodifiziert werden, beispielsweise Glas oder Quarz oder Silizium durch Vorbehandlung mit Trichlormethylsilan, Dichlordimethylsilan, Trichloroctadecylsilan, Hexamethyldisilazan oder durch Plasmaätzung bzw. Plasmapolymerisation. In bevorzugter Weise handelt es sich um Träger aus gegebenenfalls oberflächenmodifiziertem Glas, Quarzglas, Silizium, Kunststoff oder eine metallisierten Kunststoff. Weiterhin bevorzugte Träger sind optisch transparent. Alle Trägermaterialien sind weiterhin durch eine gleichmäßige Oberfläche, bevorzugt durch eine ebene Oberfläche, ausgezeichnet.

Auf solche Träger werden mit Hilfe der LB-Technik eine oder mehrere monomolekulare Schichten aufgebracht. Unter LB-Technik wird im folgenden ein Verfahren zur Übertragung von monomolekularen Schichten von einer Flüssigkeits-(Wasser-)oberfläche auf einen festen Träger nach dem Langmuir-Blodgett-Verfahren verstanden. Dazu taucht man in an sich bekannter Weise einen festen Träger mit im wesentlichen glatter Oberfläche durch einen komprimierten monomolekularen Film auf der Flüssigkeitsoberfläche und überträgt dadurch diesen Film auf den Träger.

Durch mehrmaliges Ein- und Austauchen lassen sich hierdurch Mehrschichtsysteme herstellen. Die Schicht auf der Flüssigkeitsoberfläche kann nach jedem Tauchvorgang ausgetauscht werden, so daß unterschiedliche Folgen von Schichten auf dem Träger herstellbar sind.

Das Ein- bzw. Austauchen kann senkrecht oder schräg zur Flüssigkeitsoberfläche erfolgen. Des weiteren kann gemäß der Langmuir-Schäfer-Technik der Träger auch punktuell oder mit einer Kante die Flüssigkeitsoberfläche berühren und dann auf die Flüssigkeitsoberfläche geklappt werden. Schließlich kann der Träger auch parallel auf die Flüssigkeitsoberfläche abgesenkt werden ("horizontal dipping").

Die Übertragung findet bei einer Temperatur von 5-40 °C, bevorzugt bei Raumtemperatur, statt.

Diese geordneten LB-Schichten können aus niedermolekularen und/oder polymeren Amphiphilen, bevorzugt aus polymeren Amphiphilen, bestehen und kovalent gebundene Fluoreszenzchromophore/farbstoffe und/oder amphiphile Fluoreszenzchromophore/farbstoffe, die im folgenden als $F_1$ bezeichnet werden, enthalten.

Weiterhin sind in diesen LB-Schichten funktionelle Moleküle als Rezeptoren, beispielsweise Glykolipide, Poly- und Oligonukleotide, Proteine oder Fragmente derselben, Haptene und andere, enthalten oder damit kovalent verknüpft. An diese Rezeptoren kann nun eine spezifische Bindung eines hierzu komplementären Moleküls (Ligand), wie eines Lectins, eines Antigens, eines Antikörpers und anderer, das mit einem zweiten, zu $F_1$ für einen Energietransfer passenden Fluoreszenzfarbstoff $F_2$ markiert ist, erfolgen. Im Falle der Bindung zwischen Rezeptor und Ligand soll zwischen $F_1$ und $F_2$ der sogenannte Förster-Abstand, wie er für den oben beschriebenen Energietransfer nötig ist, eingehalten werden. Diese Bedingung wird durch die Anwendung der LB-Technik gewährleistet, die eine gezielte molekulare Architektur, insbesondere in Dimensionen des hier interessierenden Bereiches von etwa 10-100 Å, gestattet. Wird das im Vorangegangenen beschriebene System nun mit Licht der Wellenlänge $\lambda_1$ angeregt, so läßt sich eine Emission des Fluoreszenzfarbstoffes $F_2$ mit einer Wellenlänge $\lambda_3$ detektieren, die als Nachweis für die Bindung des mit $F_2$ markierten Moleküls an der Sensoroberfläche, die mit $F_1$ dotiert ist, gilt. Die Anregung mit Licht der Wellenlänge $\lambda_1$ kann so erfolgen, daß $F_1$ in der LB-Schicht durch den optisch transparenten Träger hindurch in Transmission oder durch Evanescent-wave-Technik, wenn der optisch transparente Träger als Lichtleiter fungiert, oder aber durch Auflichtbestrahlung angeregt wird.

Die spezifische Wechselwirkung zwischen 2 zueinander komplementären Molekülen ist dem Fachmann auf dem Gebiet von biologisch, biochemisch und ganz besonders medizinisch (physiologisch) bedeutsamen Molekülen, etwa der oben genannten Art, bekannt. Solche Wechselwirkungen gehen letztendlich auf ionische Bindungen, Wasserstoffbrückenbindungen und van der Waalssche Kräfte zurück, die jedoch im Bereich der oben genannten Moleküle nur bei speziellen räumlichen (sterischen) Gegebenheiten wirksam werden (Schlüssel-Schloß-Theorie). Selbstverständlich kann der erfindungsgemäße Optische Biosensor auch zur Erkennung spezifischer Wechselwirkungen ohne solche speziellen räumlichen Gegebenheiten eingesetzt werden; dieser Einsatz ist insbesondere zur Überprüfung der Funktionstüchtigkeit, der Meßgenauigkeit und anderer Eigenschaften des erfindungsgemäßen Optischen Biosensors wichtig.

Der so beschriebene Sensoraufbau kann in dieser Funktion nicht nur einen in Lösung vorhandenen Analyten, der mit Fluoreszenzfarbstoff $F_2$ markiert ist, nachweisen; der Sensoraufbau kann auch genutzt werden, um in einer kompetitiven Funktionsweise einen nicht fluoreszenzmarkierten Analyten nachzuweisen. Hierzu werden bei der Präparation des Sensors die spezifisch bindenden Stellen der funktionellen Moleküle in der LB-Schicht mit fluoreszenzmarkierten, komplementär bindenden Molekülen abgesättigt. Man beobachtet dann bei Anregung mit Licht der Wellenlänge $\lambda_1$ eine maximale Fluoreszenz-Emission der Wellenlänge $\lambda_3$, deren Abnahme über einen zeitlichen Verlauf beobachtet werden kann, wenn bei Kontakt mit der zu untersuchenden Lösung die mit $F_2$ fluoreszenzmarkierten, komplementär bindenden Moleküle in einer Gleichgewichtsreaktion durch nicht fluoreszenzmarkierte, komplementär bindende Moleküle des gleichen Typs verdrängt werden.

Für den Aufbau von LB-Schichten verwendet man amphiphile Moleküle, d.h. Moleküle, die ein hydrophiles Ende (einen "Kopf") und ein hydrophobes Ende (einen "Schwanz") haben. Solche amphiphilen Moleküle können niedermolekulare Verbindungen mit einem Molekulargewicht von bis etwa 2.000 g/mol sein. In einer weiteren Variante können diese niedermolekularen Amphiphile polymerisationsfähige bzw. zur Polykondensation und Polyaddition befähigte funktionelle Gruppen enthalten, so daß nach dem Aufbau der LB-Schichten aus niedermolekularen Amphiphilen in einer nachträglichen Reaktion in der LB-Schicht diese Amphiphile zu hochmolekularen Verbindungen verknüpft werden können. Diese nachträgliche Reaktion zu hochmolekularen Verbindungen ist deshalb von Vorteil, weil LB-Schichten aus polymeren Amphiphilen höhere thermische und mechanische Stabilitäten aufweisen.

Besonders elegant lassen sich LB-Filme aus amphiphilen Polymeren herstellen, indem man die Verknüpfung der amphiphilen Einheiten herbeiführt, bevor diese dann in bekannter Weise auf der Flüssigkeitsoberfläche zu monomolekularen Schichten gespreitet werden. Die Verwendung solcher präpolymerisierter amphiphiler Polymere vermeidet so eine durch nachträgliche Polymerisation im LB-Film mögliche Störung des einmal hergestellten Ordnungszustandes.

Beispiele für polymere Amphiphile, wie sie für den erfindungsgemäßen optischen Biosensor geeignet sind, sind $\alpha$-Olefin-Maleinsäureanhydrid-Copolymere (British Polymer Journal 17 (1985), 368 ff; J. Macromol. Sci.-Phys. B 23 (1985), 549-573), Polyoctadecyl-methacrylat, Polyvinylstearat (J. Coll. Interface Sci. 86 (1982), 485), Polyvinylphospholipide (Angew. Chem. 100 (1988), 117-162), Cellulose-tristearat, amphiphile Polyamide (DE-OS 3 830 325) und Acrylamid-Copolymere. In bevorzugter Weise für die Herstellung stabiler LB-Schichten sind Polyurethane gemäß DE-OS 3 827 438 und Polyester gemäß DE-OS 3 830 862 geeignet. Unter den polymeren Amphiphilen sei weiterhin ganz besonders auf statistische Poly-(alkylmethacrylat)-Copolymere des folgenden Typs verwiesen, die in ihrer Zusammensetzung breit variierbar sind:

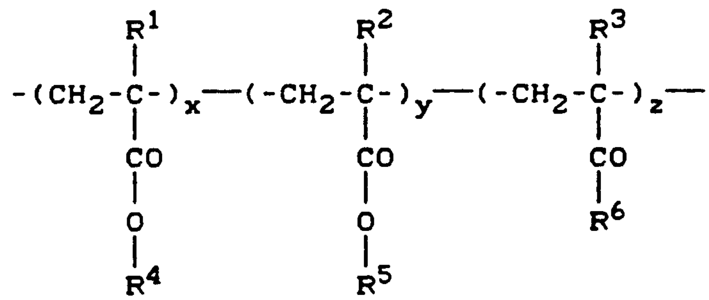 (I),

in der

- $R^1$, $R^2$ und $R^3$ unabhängig voneinander Wasserstoff oder Methyl darstellen,
- $R^4$ geradkettiges $C_{14}$-$C_{22}$-Alkyl ist,
- $R^5$ das Wasserstoff-, Natrium- oder Kaliumion ist oder eine der Gruppen $-CH_2-CH_2OH$, $-CH_2-CH_2-NH$-tert.-butyl, $-CH_2-CH_2-N(CH_3)_2$,

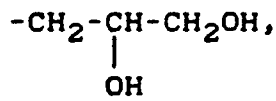

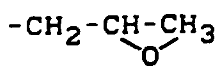 oder 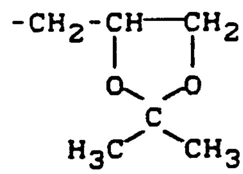

darstellt,

- $R^6$ ein dem Fachmann bekannter Fluoreszenzchromophor ist, wie er weiter unten dargestellt ist, und
- x einen Wert von 0,2-1,
- y einen Wert von 0-0,8 und
- z einen Wert von 0-0,2 annimmt, wobei die Summe x + y + z = 1 beträgt.

In bevorzugter Weise sind x und y etwa gleich.

Beispiele für Polymere der Formel (I) sind die folgenden:

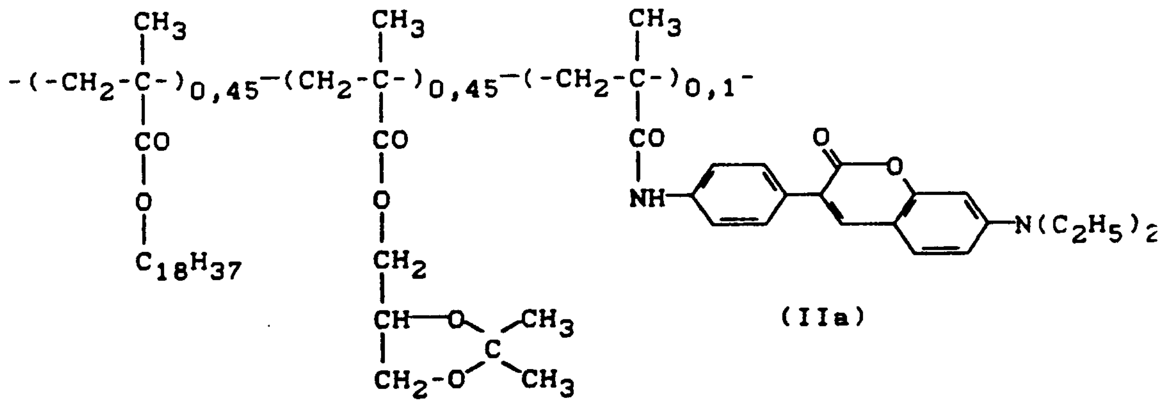

(IIa)

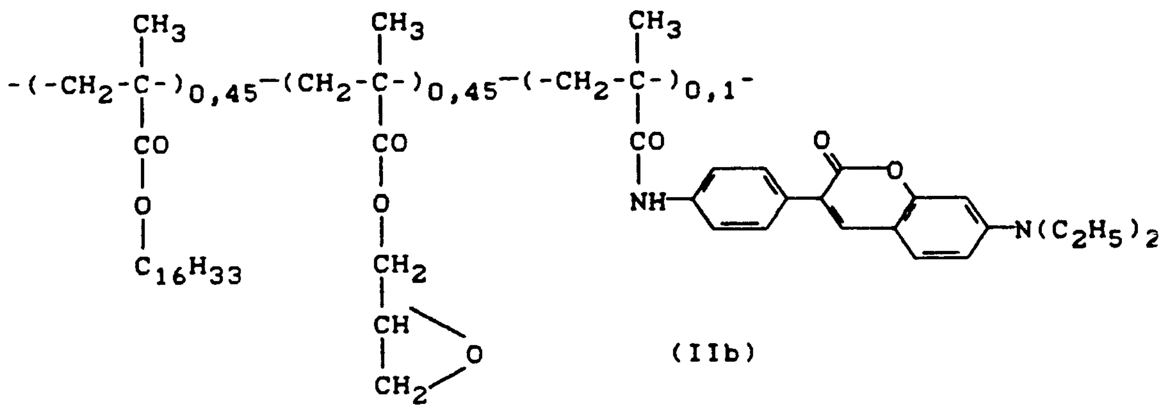

(IIb)

Bei den hier beispielhaft genannten Substanzen für LB-Mono- und Multischichten ist der Fluoreszenzchromophor an das amphiphile Polymer kovalent geknüpft. Wenngleich diese Anordnung die größtmögliche Stabilität der $F_1$ enthaltenden LB-Schichten ermöglicht, ist es aber auch möglich, $F_1$ enthaltende LB-Schichten durch Cospreiten eines amphiphilen Polymers mit amphiphilen Fluoreszenzfarbstoffen auf der Wasseroberfläche vor dem Beschichtungsvorgang zu erzielen. Als Beispiele für solche amphiphile Fluoreszenzfarbstoffe, die mit amphiphilen Polymeren, welche keine Chromophore enthalten, gemeinsam eingesetzt werden können, sind etwa Cyaninfarbstoffe der Typen

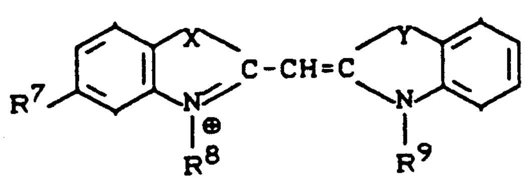

(IIIa) und

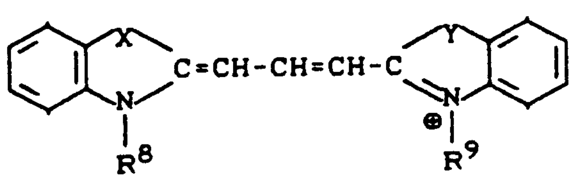

(IIIb),

in denen

X und Y unabhängig voneinander für Sauerstoff, Schwefel oder Selen oder $C(CH_3)_2$ stehen,

$R^7$ Wasserstoff oder Methyl bedeutet und

$R^8$ und $R^9$ unabhängig voneinander für geradkettiges $C_1$-$C_{22}$-Alkyl stehen.

Weitere Beispiele für dem Fachmann grundsätzlich bekannte und erfindungsgemäß einsetzbare Fluoreszenzfarbstoffe sind Farbstoffe der folgenden Typen:

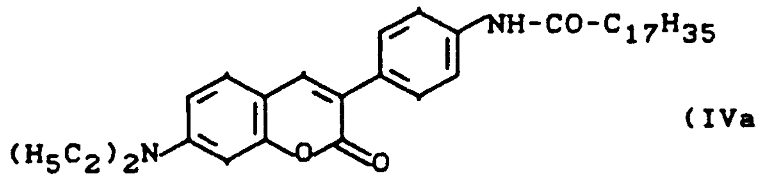

(IVa)

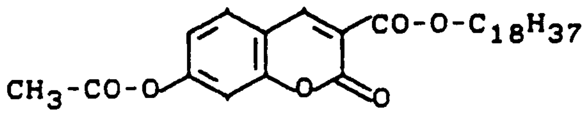

(IVb)

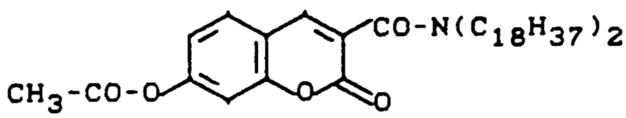

(IVc)

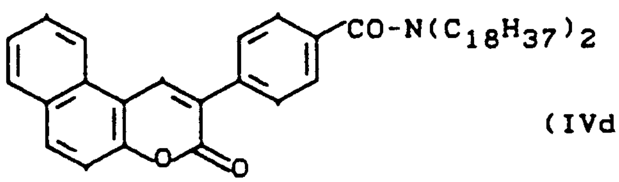

(IVd)

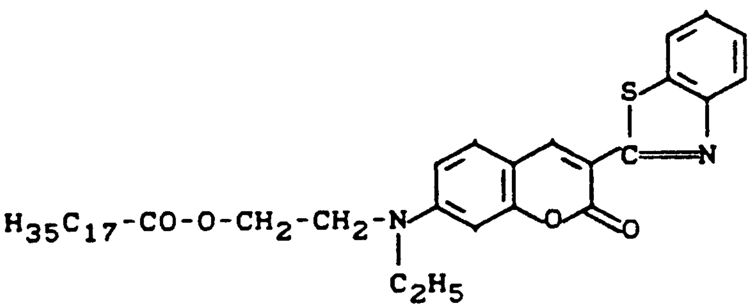

(IVe)

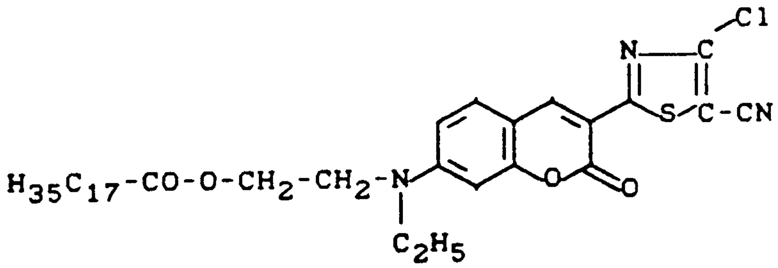

(IVf)

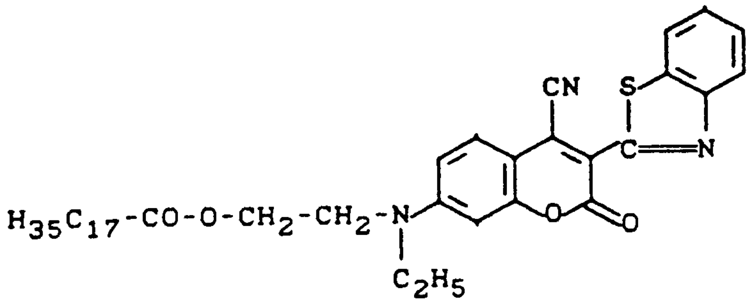

(IVg)

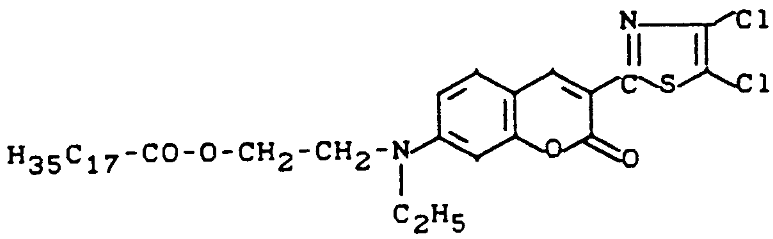

(IVh)

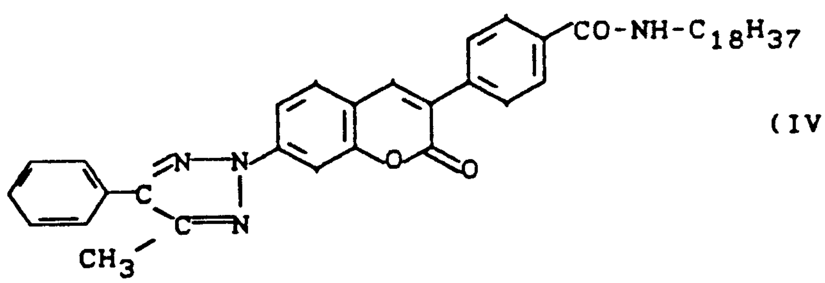

(IVi)

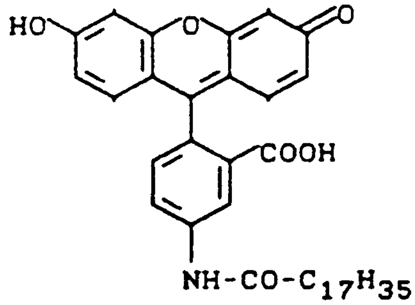

(IVj)

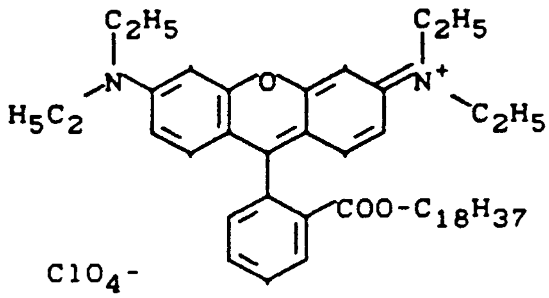

(IVk)

Diese Aufzählung ist nur beispielhaft. Weitere amphiphile Fluoreszenzfarbstoffe sind beschrieben in der Monographie Physical Methods of Chemistry, Vol. 1, Part. 3B, S. 577 ff, John Wiley, New York 1972. Will man solche amphiphile Fluoreszenzfarbstoffe in LB-Schichten einbringen, muß auf eine gleichmäßige Verteilung des Farbstoffs in der gesamten Schicht geachtet werden. So ist zu vermeiden, daß die Übertragung einzelner Schichten je nach Temperatur (typisch 5-40°, vorzugsweise etwa 20°C) bei einem solchen angelegten Schub erfolgt, bei dem ein Coexistenzbereich der festanalogen- und flüssiganalogen Phase durchlaufen wird. Dies ist wichtig, da der amphiphile Fluoreszenzfarbstoff in der Regel in den beiden Phasen nicht die gleiche Löslichkeit besitzt und so inhomogene, für die Sensoranwendung weniger geeignete Schichten gebildet werden. Diese Erscheinung ist bei LB-Schichten aus niedermolekularen Substanzen bekannt (Angew. Chem. 100 (1988), 750); auch bei polymerisierten Phospholipiden ist diese Erscheinung beobachtet worden (Polymer Sci. 267 (1989), 97-107).

Bei der Herstellung erfindungsgemäßer optischer Biosensoren wurde überraschenderweise gefunden, daß LB-Schichten aus Polymeren der Formel (II) beim Anlegen von Schüben bis zum Zusammenbruch der LB-Schicht bei >45 mN/m Schub nicht zur Ausbildung phasenseparierter Domänen neigen. Dies gilt neben Polymeren der Formel (II) auch für eine Mischung aus einem Polymer vom Typ der Formel (V) und einem Farbstoff, beispielsweise der Formel (IVa), wobei das Polymer vom Typ der Formel (V) als "Matrix" anzusehen ist, in welcher sich 0,1 bis 25 Mol-% des amphiphilen Farbstoffs aufhalten können, wobei beim Polymer die Wiederholungseinheiten zur Berechnung der Molprozente herangezogen werden:

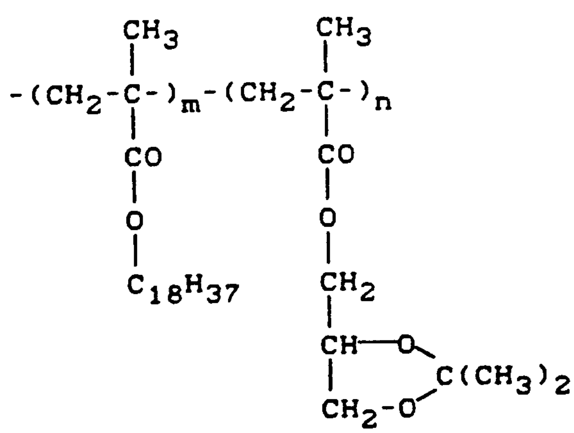

(V),

worin

- m Werte von 0,25-1 und
- n Werte von 1-m annimmt.

In bevorzugter Weise nimmt m Werte von 0,4-0,6 an.

Derart hergestellte LB-Schichten zeigen sowohl auf Wasser als Subphase als auch nach dem Übertragen auf einen festen Träger lichtmikroskopisch homogene Schichten ohne Störungen und sind für die erfindungsgemäßen Biosensoren besonders geeignet.

Bei Systemen mit phasenseparierten Domänen kann aber auch eine hohe Empfindlichkeit des erfindungsgemäßen optischen Biosensors erreicht werden, wenn Fluoreszenzfarbstoffe $F_1$ als Donor in LB-Schichten verwendet werden, die aufgrund ihres speziellen Verhaltens Aggregate mit fluoreszenzspektroskopischen Eigenschaften bilden, die von denen des monomeren Farbstoffs sehr verschieden sind und die sich in der Regel durch eine entsprechend schärfere und intensivere Absorptionsbande und entsprechend schärfere und intensivere Fluoreszenz-Emissionsbande auszeichnen. Solche Aggregate sind dem Fachmann als J-Aggregate oder Scheibe-Aggregate bekannt (Physical Methods of Chemistry, Vol. 1, Part. 3B, S. 579, John Wiley, New York 1972). Mit dem speziellen Verhalten solcher J-Aggregate kann zum einen eine sehr hohe Farbstoffdichte $F_1$ in den LB-Schichten und zum anderen aufgrund der starken Absorption von Licht der Wellenlänge $\lambda_1$ eine hohe Energiedichte, die nach der Theorie von Förster auf entsprechende Moleküle $F_2$ übertragen werden kann, erreicht werden. Die geringe Halbwertsbreite der Emissionsbande bedeutet sowohl einen Verstärkungseffekt des Meßsignals, als auch eine Verminderung der Störstrahlung durch geringere Überlappung der Emissionen von $F_1$ mit $F_2$.

Fluoreszenzfarbstoffe, die in LB-Schichten in der Lage sind, J-Aggregate zu bilden, sind in der obengenannten Literatur beschrieben worden. Beispielhaft seien genannt: Cyaninfarbstoffe und Merocyanine.

Der Einbau funktioneller Moleküle in die den Fluoreszenzfarbstoff $F_1$ enthaltende LB-Schicht kann auf unterschiedliche Art und Weise erfolgen:

- Das funktionelle Molekül kann kovalent, gegebenenfalls unter Verwendung von Spacer-Molekülen, an die LB-Schicht geknüpft sein, sei es von Beginn des Spreitungsvorganges auf der Wasseroberfläche an oder durch eine nachträgliche Kupplungsreaktion an die LB-Schicht entweder auf der Subphase oder nach Aufbringen der LB-Schicht auf einen festen Träger.
- Das funktionelle Molekül kann als Amphiphil mitgespreitet und so physikalisch mit "Anker" in die LB-Schicht eingebaut werden.

Für beide Einbauvarianten sind aus der Literatur Methoden bekannt. Beispielsweise kann die Verknüpfung biologischer funktioneller Gruppen an LB-Schichten auf feste Träger in analoger Weise zu den aus der Biochemie dem Fachmann bekannten Immobilisierungsmethoden erfolgen (Methods in Enzymology, Vol. 135 und Vol. 136 (1987)). Mit langen Alkylketten versehene Moleküle werden in DE-OS 3 546 150 als Membrananker-Wirkstoffkonjugate in großer Auswahl genannt und können durch Cospreiten auf der Subphase in den LB-Film eingebaut werden. Als ein Beispiel für solche amphiphile funktionelle Moleküle seien Glykolipide, beispielsweise Ceramide, genannt. Weitere Beispiele sind Antikörper/Antigen-Systeme sowie komplementäre Nucleotidsequenzen. Eine große Anzahl solcher Beispiele ist dem Fachmann bekannt (Angew. Chem. 100 (1988), 117-162).

Entscheidend für die Steigerung der Empfindlichkeit des Sensorsystems ist ein möglichst hohes Verhältnis $F_1$:$F_2$ innerhalb des "Förster-Radius" und damit eine entsprechende Verstärkung des Fluoreszenzsignals eines mit $F_2$ markierten Moleküls nach erfolgter Bindung an eine mit $F_1$ dotierte Oberfläche. Es ist demnach vorteilhaft, möglichst viele $F_1$-Chromophore in den obersten LB-Schichten, insbesondere in den obersten vier Lagen einzubringen. In besonders bevorzugter Weise ist der Farbstoff $F_1$ in mindestens einer der beiden oberen Lagen angeordnet.

Wenngleich in der Fluoreszenzspektroskopie üblicherweise Konzentrationen an Fluoreszenzfarbstoff unter 1 % verwendet werden, um Wechselwirkungen zwischen den einzelnen Farbstoffmolekülen und damit Änderungen ihres Fluoreszenzverhaltens zu vermeiden, ist es im erfindungsgemäßen optischen Biosensor dennoch vorteilhaft, den Fluoreszenzfarbstoff $F_1$ in hohen Konzentrationen in die LB-Schichten einzubringen. Insbesondere polymere amphiphile Fluoreszenzfarbstoffe zeigen eine geringere Tendenz zur Eigenlöschung und Excimerenbildung bei Farbstoffkonzentration von 0,1-25 Mol-%. Der gleiche Konzentrationsbereich erweist sich auch als vorteilhaft für den Fall, daß isolierte Chromophore gleichmäßig in der LB-Schicht verteilt sein sollen. Im besonderen Fall der Scheibe-Aggregate ist hingegen die Assoziation von Chromophoren erwünscht. Diese Assoziation tritt bevorzugt bei Farbstoffkonzentrationen oberhalb von 25 Mol-% bis zu 100 Mol-% (ohne polymere Matrix) ein.

Der erfindungsgemäße optische Biosensor zeigt weiterhin den Vorteil, das unabhängig von den in die Schichten der festen Phase eingebrachten funktionellen Molekülen der für das Prinzip des Energietransfers erforderliche Farbstoff $F_1$ frei wählbar in weiten spektralen Bereichen in die LB-Schicht eingebracht werden kann. Dies bedeutet, daß zum einen das funktionelle Molekül nicht spezifisch mit $F_1$ markiert werden muß und zum anderen der spektrale Bereich von $F_1$ optimal für einen Energietransfer auf den als Marker

verwendeten Farbstoff $F_2$ abgestimmt werden kann. Beispiele von Paaren sind:

| $F_1$ | $F_2$ |
|---|---|
| a) Polymer (IIa) | TRITC |
| b) Cyanin (IIIb) mit X = Y = O, $R^7$ = H, $R^8$ = $R^9$ = $C_{18}H_{37}$ | TRITC oder FITC |
| c) Cyanin (IIIa) mit X = Se, Y = S, $R^7$ = $CH_3$, $R^8$ = $R^9$ = $C_{18}H_{37}$ | FITC oder TRITC |
| TRITC = Tetramethylrhodamin-isothiocyanat<br>FITC = Fluorescein-isothiocyanat. | |

Im erfindungsgemäßen optischen Biosensor wird wie beschrieben die Steigerung der Empfindlichkeit des fluoreszenzspektroskopischen Nachweises dadurch erreicht, das eine möglichst hohe Farbstoffkonzentration $F_1$ in die LB-Schicht eingebracht wird und somit mehrere Moleküle $F_1$ in den für den Energietransfer auf ein an die Schicht gebundenes Molekül $F_2$ notwendigen "Förster-Radius" gelangen. Dieser Aufbau, eine möglichst hohe Farbstoffdichte $F_1$ im LB-Schichtsystem neben dem Rezeptor einzubringen, erlaubt im Gegensatz zu den bisher bekannten, auf Energietransfer beruhenden Nachweismethoden eine viel günstigere Ausnutzung dieses Meßprinzips und damit eine deutlich erhöhte Empfindlichkeit, weil pro Rezeptor-Molekül eine viel höhere Anzahl an Farbstoffmolekülen vorhanden sein kann als bei einer direkten Fluoreszenzmarkierung des Rezeptor-Moleküls.

Aus der Nutzung aller innerhalb des Förster-Radius von $F_2$ liegenden Farbstoffmoleküle $F_1$ folgt auch, das nicht nur die laterale Verteilung von $F_1$ innerhalb der obersten LB-Schichten von entscheidender Bedeutung ist, sondern auch die Konzentration von $F_1$ in den darunterliegenden Schichten. Aus diesem Grund beschränkt sich das Meßprinzip auf Schichten bis zu etwa 100 Å wirksamer Schichtdicke, denn darunterliegende Moleküle $F_1$ können nach Anregung durch Licht ihre Energie nicht mehr in ausreichendem Maße auf den dann zu weit entfernten Farbstoff $F_2$ übertragen und würden das zu detektierende Signal, nämlich die Lichtemission der Wellenlänge $\lambda_3$ des durch Transfer angeregten Farbstoffs $F_2$, durch ihre eigene Fluoreszenz mit der Wellenlänge $\lambda_2$ überwiegend stören und die Empfindlichkeit des Nachweises unnötig herabsetzen.

Aus diesem Grund eignen sich für die Herstellung dünner Schichten (100 Å oder darunter), die $F_1$ enthalten, einzig die LB-Schicht-Technologie und die Chemisorption. Nämlich bereits die in der Dünnschichttechnologie verbreitete Methode des Spin-coating bereitet mit minimalen Schichtdicken von 200 bis 500 Å Probleme. Gegenüber dem Aufbringen dünner Schichten durch Chemisorption besitzt die LB-Technik den Vorteil, daß die Schichten in ihrer Zusammensetzung sehr definiert eingestellt werden können, was für die Herstellung reproduzierbarer Oberflächen für Sensoren von entscheidender Bedeutung ist.

Der Donorfarbstoff $F_1$ und die bereits genannten aktiven Stellen zur Anbindung eines Biomoleküls können sich dabei auch in verschiedenen, übereinander liegenden LB-Schichten befinden. Die Gesamtzahl der für das Sensorprinzip wirksamen LB-Schichten bewegt sich im Zahlenbereich von 1 bis 10.

Zum erfindungsgemäßen optischen Biosensor gehören weiterhin mobile, fluoreszierende Moleküle, die die Farbstoffkomponente $F_2$ enthalten und die an die fest in der LB-Schicht verankerten Rezeptormoleküle reversibel gebunden werden. Lediglich im einfachsten Fall, nämlich der Bestimmung eines selbstfluoreszierenden und damit als $F_2$ wirkenden Analyten, ist diese Komponente nicht erforderlich, da $F_2$ und Ligand identisch sind und den Analyten darstellen. Zum einen können die Bindungsstellen der Rezeptoren auf der LB-Schicht durch fluoreszenzmarkierte Derivate oder Analoga des Analytenmoleküls abgesättigt sein, die dann im Kontakt mit der Probenlösung vom Analyten kompetitiv verdrängt werden können. Zum anderen ist jedoch auch ein Sandwich-Immunoassay möglich, bei dem eine zweite Art von Rezeptoren, beispielsweise Antikörpern, entweder an den Komplex zwischen erstem Rezeptor und dem Analyten oder an einen Molekülbereich des Analyten, die an der Bindung zum ersten Rezeptor nicht beteiligt ist, binden. Diese Methoden der Festphasen-Immunoassays sind prinzipiell Stand der Technik und beispielsweise in der Monographie P. Tijssen, Practice and Theory of Enzyme Immunoassays (R.H. Burdon, Ph.H. van Knippenberg, Herausgeber) Elsevier, Amsterdam 1985, dargestellt.

Beispiel 1

Herstellung eines amphiphilen Fluoreszenzfarbstoffs

Zu 1,53 g (5 mmol) 7-Diethylamino-3-(p-aminophenyl)cumarin und 0,61 g (5 mmol) Triethylamin in 10 ml trockenem Chloroform wurden unter Eisbadkühlung 1,51 g (5 mmol) Octadecansäurechlorid in 5 ml

trockenem Chloroform getropft. Man ließ eine Stunde bei 0-5 ° C und 5 Stunden bei Raumtemperatur nachrühren. Der Ansatz wurde zunächst mit verdünnter Natronlauge, zuletzt mit Wasser gewaschen. Das Rohprodukt wurde zweimal aus Chloroform mit Petrolether 60/70 ° C gefällt.

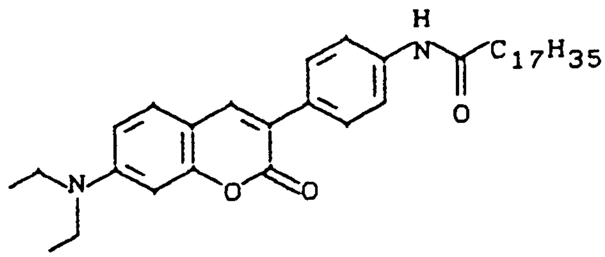

(IVa)

Es wurden 64 % der theoretischen Ausbeute des Produktes der obigen Formel mit einem Schmelzpunkt von 159 ° C erhalten.

$^1$H-NMR ($CDCl_3$, int. TMS): $\delta$ = 7.66-6.52 (Multiplett, aromatische Protonen); 3.42 (-C$\underline{H}_2$$CH_3$); 2.35 (-COC$\underline{H}_2$$C_{16}H_{33}$) 1.22 (-$CH_2$C$\underline{H}_3$); 1,7-0,8 (-$COCH_2C_{16}$$\underline{H}_{33}$).

In analoger Weise wurden auch die weiter vorn aufgeführten Verbindungen IVb bis IVi hergestellt. Einige spektroskopische Daten sind in Tab. 1 zusammengestellt.

Tabelle 1

| Spektroskopische Daten amphiphiler Farbstoffe | | | |
|---|---|---|---|
| Formel | Spektroskopische Daten in $CH_2Cl_2$ | | |
| | Exc. max λ (nm) | Em. max λ (nm) | Δ Stokes (nm) |
| IVa | 406 | 476 | 70 |
| IVe | 528 | 549 | 21 |
| IVf | 475 | 511 | 36 |
| IVg | 531 | 585 | 54 |
| IVh | 460 | 495 | 35 |
| IVi | 368 | 452 | 84 |

Beispiel 2

Herstellung eines polymerisierbaren Fluoreszenzfarbstoffs

Zu 1,53 g (5 mmol) 7-Diethylamino-3-(p-aminophenyl)cumarin und 0,61 g (5 mmol) Triethylamin in 10 ml trockenem Chloroform wurden unter Eisbadkühlung 0,52 g (5 mmol) Methacrylsäurechlorid in 5 ml trockenem Chloroform getropft. Man ließ eine Stunde bei 0-5 ° C und 5 Stunden bei Raumtemperatur nachrühren. Der Ansatz wurde zunächst mit Natronlauge, zuletzt mit Wasser bis zur Salzfreiheit gewaschen und zur Trockne eingeengt.

Ausbeute: 1,6 g entsprechend einer theoretischen Ausbeute von 86 % des Produktes der weiter unten (Tab. 2) aufgeführten Formel VIa mit einem Schmelzpunkt von 193-195 ° C.

$^1$H-NMR ($CDCl_3$, int. TMS): $\delta$ = 9.43 (NH), 7.77-6.49 (Multiplett, arom. Protonen); 5.86 und 5.47 ($H_2C$=); 3.44 (-C$\underline{H}_2$$CH_3$); 2.04 (=C-$CH_3$); 1.22 (-$CH_2$C$\underline{H}_3$).

In analoger Weise wurden auch die ebenfalls in Tab. 2 aufgeführten Verbindungen VIb bis VIf hergestellt. Einige spektroskopische Daten sind in Tab. 2 zusammengestellt

Tabelle 2:

Spektroskopische Daten polymerisierbarer Fluoreszenzfarbstoffe

| Formel | Spektroskopische Daten in $CH_2Cl_2$ | | |
|---|---|---|---|
| | Exc.$_{max}$ λ (nm) | Em.$_{max}$ λ (nm) | ΔStokes (nm) |
| 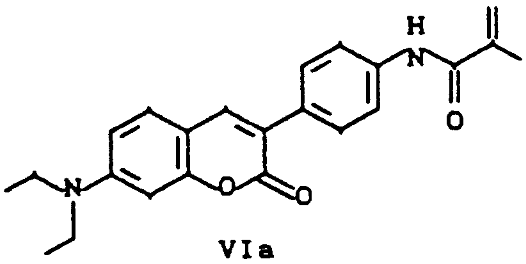 VIa | 395 | 475 | 80 |

| Formel -Fortsetzung - | Spektroskopische Daten in $CH_2Cl_2$ | | |
|---|---|---|---|
| | Exc. $\lambda_{max}$ (nm) | Em. $\lambda_{max}$ (nm) | ΔStokes (nm) |
| 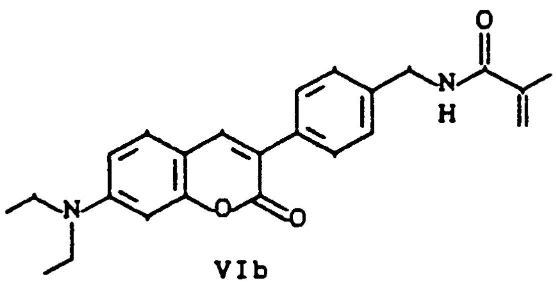 VIb | 401 | 475 | 74 |
| 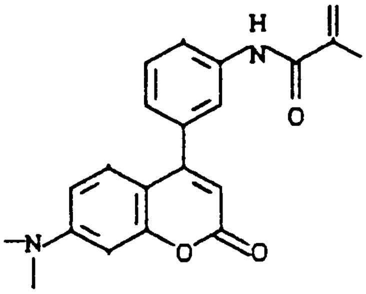 (VIc) | 382 | 491 | 110 |
| 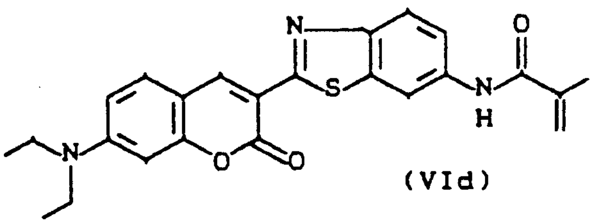 (VId) | 456 | 507 | 51 |
| 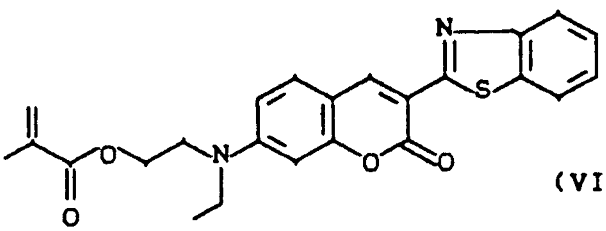 (VIe) | 528 | 546 | 17 |

| Formel -Fortsetzung - | Spektroskopische Daten in $CH_2Cl_2$ Exc. max. λ (nm) | Em. max. λ (nm) | ΔStokes (nm) |
|---|---|---|---|
| 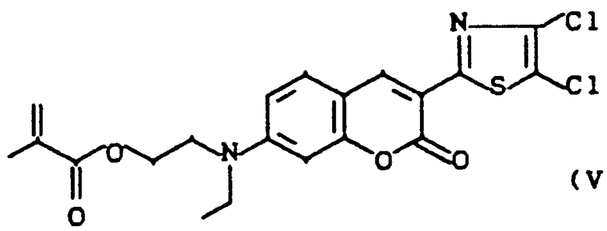 (VIf) | 450 | 496 | 46 |

Beispiel 3

Herstellung eines Fluoreszenzfarbstoff enthaltenden Polymeren

6,77 g (20 mmol) Octadecylmethacrylat, 4,00 g (20 mmol) (2,2-Dimethyl-1,3-dioxolan-4-yl)-methylenmethacrylat und 1,51 g (4 mmol) des Farbstoffmonomeren des Beispiels 2 wurden in 90 ml absolutem Dioxan gelöst und nach Zugabe von 1,44 g (0,2 Mol-%) Azo-bis(isobutyronitril) unter Rühren auf 65-70 °C erwärmt und 16 Stunden lang bei dieser Temperatur gehalten. Nach Abkühlen wurde aus der Reaktionslösung das Polymer durch Eintragen in Wasser ausgefällt. Das Polymer wurde durch zweimaliges Lösen in Chloroform und Fällen in Methanol gereinigt.

Es wurden 3,93 g eines gelbgrünen Polymers erhalten, das durch Gelpermeationschromatographie in $CH_2Cl_2$ charakterisiert wurde. Gleichzeitige Detektion von Brechungsindex und UV-Spektroskopie lieferte identische Molekulargewichtsverteilungskurven, so das ein gleichmäßiger Einbau des Fluoreszenzfarbstoffs in das Polymer gewährleistet war. Gegenüber einer Polystyrol-Eichung ergaben sich rechnerisch Molmassenwerte $M_n$ = 64.000 und $M_w$ = 1.290.000 entsprechend einer Uneinheitlichkeit von 18.1.

Nach dieser allgemeinen präparativen Vorschrift wurden alle Methacrylat-Copolymere hergestellt.

Beispiel 4

Herstellung eines Schichtelements mit Fluoreszenzfarbstoff

a) Polymerer Farbstoff
Ein durch Behandlung mit $H_2O_2/H_2SO_4$ gereinigter Objektträger aus Floatglas wurde bis zu einer Tiefe von 30 mm in die wäßrige Subphase einer Langmuir-Filmwaage (KSV 2200) bei 20 °C eingetaucht. 150 µl einer Lösung der Verbindung der Formel (IIa) in Chloroform (1 mg/ml) wurden auf die Wasseroberfläche aufgespreitet. Nach dem Zusammenschieben der Schicht auf einen Oberflächendruck von 25 mN/m wurden durch sukzessives Aus- und Eintauchen (Tauchgeschwindigkeit: 10 mm/min) des Objektträgers drei monomolekulare Lagen Polymer übertragen. Die letzte Schicht wurde dabei beim Austauchen übertragen. Der Träger wurde anschließend an der Luft getrocknet. Eine Seite des Trägers wurde durch Reinigung mit Chloroform von der Farbstoffschicht befreit.

b) Polymer mit dispergiertem monomeren Farbstoff Anstelle der Lösung des farbstoffhaltigen Polymers der Formel (IIa) wurde eine Mischung des Polymers der Formel (V), 1 mg/ml, und des monomeren amphiphilen Farbstoffs der Formel (IIIb) mit X=Y=O, n=18, $R^7$=H, $R^8$=$R^9$=$C_{18}H_{37}$, 1 mg/ml, im Verhältnis 19:1 verwendet.

c) Polymer mit dispergiertem Farbstoff, der Scheibe-Aggregate bildet.

Aufgezogen wurde eine Mischung des Polymers der Formel (V), 1 mg/ml, und des Farbstoffs der Formel (IIIb) mit X=Se, Y=S, $R^8$=$R^9$=$C_{18}H_{37}$, $R^2$=$CH_3$, 1 mg/ml, im Gewichtsverhältnis von 1:1.

Beispiel 5a

Adsorption von Fluoreszenzfarbstoffen an ein Schichtelement und Beobachtung von Fluoreszenz-Energietransfer

Ein nach Beispiel 4 hergestelltes Schichtelement wurde in eine Lösung von $10^{-7}$ mol/l Fluorescein in Phosphatpuffer, pH 7.0, 5 min lang getaucht. Vor und nach dem Versuch wurde ein Fluoreszenzspektrum aufgenommen. Das Emissionsspektrum verschob sich zum Maximum von Fluorescein hin.

Beispiel 5b

Herstellung eines Schichtelements mit Fluoreszenzfarbstoff:

Ein durch Ultraschallbehandlung in einer wäßrigen Detergenslösung gereinigter und anschließend durch Ultraschallbehandlung mit reinem Wasser und weiterer Ultraschallbehandlung (5 min.) in ca. $5 \cdot 10^{-2}$ n NaOH sowie durch Abspritzen mit reinem Wasser unter einem Druck von 5 atm gespülter und danach getrockneter Glasträger wurde im Exsikkator durch Einwirkung von Hexamethyldisilazan hydrophobiert (30 min. bei 60 °C im Wasserstrahlpumpenvakuum). Der Glasträger wird nach dieser Behandlung kurz in Wasser getaucht und seine Oberfläche nach dem Herausnehmen aus dem Wasser sorgfältig abgesaugt. Auf diesen Träger wurden nach der LB-Technik durch Ein- und Austauchen des Trägers zwei Schichten von Cadmiumarachidat übertragen.

Die darauf folgende Schicht des Fluoreszenzfarbstoffs (VII) = (IIIa) mit X = Y = O, $R^7$ = H, $R^8$ = $R^9$ = $C_{18}H_{37}$ wurde in unterschiedlicher Organisation, i) als Monomere des Farbstoffs und ii) als Scheibe-Aggregate (J-Aggregate) des Farbstoffs präpariert und übertragen.

i) Monomere des Farbstoffs (VII)

Ein monomolekularer Film auf Wasser wurde durch Spreiten einer Lösung erzeugt, die (VII), Methylarachidat, Arachinsäure und Hexadekan im molaren Verhältnis 1:2:18:20 in Chloroform enthält.

ii) Scheibe-Aggregate des Farbstoffs (VII)

Ein monomolekularer Film auf Wasser wurde durch Spreiten einer Lösung von (VII) und Hexadekan im molaren Verhältnis 1:1 in Chloroform erzeugt.

Der folgende Aufbau des Schichtelements ist für Monomere und Scheibe-Aggregate identisch. Nach Kompression des Films auf einen Schub von 20 mN/m und Lagerung von 10 min. bei konstantem Schub wurde der Film durch nahezu horizontales Kontaktieren des Trägers mit dem Monofilm auf den Träger übertragen. Der Träger wurde dann vollständig in das Wasser eingetaucht, der restliche Film des Farbstoffs wurde entfernt, und ein Monofilm von Stearinsäure durch Spreiten einer $10^{-3}$ m Lösung in Chloroform und Kompression auf 20 mN/m gebildet. Der Träger wurde dann durch senkrechtes Austauchen mit einer Schicht von Stearinsäure bedeckt. Abschließend wurde der Träger mit einer gemischten Schicht von Dioktadecyl-dimethyl-ammoniumbromid und Methylstearat im Molverhältnis 1:1 durch nahezu horizontales Kontaktieren und vollständiges Eintauchen des Trägers beschichtet und unter Wasser mit einem Küvettenkörper in einer dem Fachmann bekannten Weise (siehe P.Fromherz, Biochim. Biophys. Acta. 323 (1973) 326-334) zu einer Fluoreszenzküvette zusammengebaut.

Beispiel 5c

Ein nach Beispiel 5b hergestelltes Schichtelement wurde durch Austausch des wäßrigen Mediums ohne Belüftung der Oberfläche des Schichtelements in Kontakt mit einer wäßrigen Lösung des Analyten (VIII) (Formel siehe unten) in einem $10^{-4}$ m Phosphatpuffer, pH = 7,0, gebracht. Infolge Bindung des Farbstoffs an die Oberfläche des Schichtelements wird die Fluoreszenzintensität von (VII) im Fall der Scheibe-Aggregate in Abhängigkeit von der Konzentration des Analyten in der angrenzenden Lösung und der Zeit nach Herstellen des Kontakts erniedrigt. Im Fall einer $10^{-7}$ m Lösung von (VIII) beträgt die Intensität der Emission bei 404 nm und Anregung bei 366 nm nach 90 min. 33 % der Intensität in Abwesenheit von (VIII), im Fall einer $10^{-10}$ m Lösung 85 %.

Erwartungsgemäß wird eine deutliche Abstandsabhängigkeit dieses Löscheffekts beobachtet, wenn durch Einbau einer Doppelschicht von Cadmiumstearat zwischen der Stearinsäureschicht, die sich im Kontakt mit der Farbstoffschicht von der Oberfläche des Schichtelements vergrößert wird. Die Intensität der Emission des Scheibe-Aggregates bei 404 nm beträgt für eine $10^{-7}$ m Lösung von (VIII) dann 90 % der bei Abwesenheit von (VIII) beobachteten Intensität.

Die Bindung des Analyten (VIII) an die Oberfläche des Schichtelements läßt sich auch durch Messung der Fluoreszenz von (VIII) bei 510 nm nachweisen. Eine direkte Anregung (Emission des Analyten) ist bei 470 nm möglich, während die Anregung von (VII) und nachfolgende Energieübertragung zu einer maximalen Emission des Analyten führt, falls sie bei 366 nm (Monomere) bzw. 402 nm (Scheibe-Aggregate) erfolgt. Das Verhältnis der Fluoreszenzintensitäten bei 510 nm nach indirekter Anregung und Energietransfer ($I_{VII}$) und bei direkter Anregung des gebundenen Analyten ($I_A$) ist der Verstärkungsfaktor und kann aus dem Anregungsspektrum der Emission bei 510 nm bestimmt werden. Man findet für

a) Monomere:

$I_{VII}/I_A$ = 3 für $10^{-7}$ m Lösung von (VIII)
$I_{VII}/I_A$ = 35 für $10^{-8}$ m Lösung von (VIII)

b) Scheibe-Aggregate:

$I_{VII}/I_A$ = 380 für $10^{-8}$ m Lösung von (VIII)

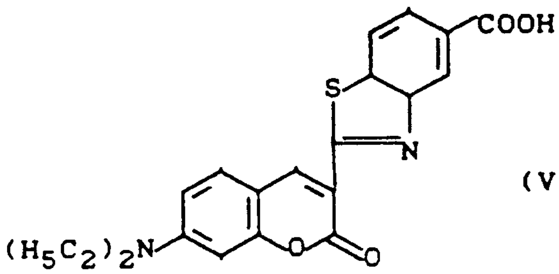

(VIII)

Beispiel 6

Adsorption von fluoreszenzmarkiertem Protein an ein Schichtelement und Beobachtung von Fluoreszenz-Energietransfer

Ein nach Beispiel 4 hergestelltes Schichtelement wurde mit 50 µl einer Lösung von mit Tetramethylrhodamin-isothiocyanat (TRITC) markiertem Lectin Concanavalin A (1 mg/ml) betropft und ein zweiter, unbehandelter Objektträger gleicher Größe derart angepreßt, das sich die Flüssigkeit gleichmäßig und ohne Luftblasen auf der Langmuir-Blodgett(LB)-Schicht verteilte. Nach einer Stunde Einwirkungszeit wurden beide Träger getrennt und der beschichtete dreimal mit wäßrigem Phosphatpuffer, 10 mmol/l, pH 6.8, gewaschen. Anschließend wurde ein Fluoreszenzspektrum gemessen und mit dem eines nicht mit Protein behandelten Schichtelements verglichen. Man erkannte eine zusätzliche Bande der TRITC-Emission. Wurden über die farbstoffhaltige LB-Schicht zwei bis sechs Farbstoff-freie Lagen aufgebracht, so sinkt die Intensität dieser Bande in Abhängigkeit von der Schichtdicke bis auf Null.

Beispiel 7 (zum Vergleich)

Herstellung von Schichtelementen mit alternativen Techniken

a) Ausstrich-Technik

50 µl einer Lösung des Polymers (IIa), 1 mg/ml, in Chloroform wurden auf einen Objektträger gebracht. Mit Hilfe eines zweiten Objektträgers wurde die Farbstofflösung möglichst gleichmäßig auf dem ersten ausgestrichen. An diese Farbstofflage wurden dann, wie in Beispiel 6 beschrieben, 50 µl TRITC-ConA adsorbiert und die Fluoreszenz gemessen. Neben der sehr intensiven Bande von (IIa) ist die Fluoreszenz des TRITC nicht eindeutig nachzuweisen.

b) Lackschleuder-Technik (Spin-Coating)

0,0193 bis 0,244 mg des Polymers (IIa) wurden in 0,25 bis 1,5 ml Chloroform oder Dimethylformamid gelöst und mit Hilfe eines Spin-Coaters auf einen gereinigten Glasträger von 10 cm Durchmesser aufgegeben. Fluoreszenzmessungen der erhaltenen Glasplättchen zeigten eine sehr heterogene Verteilung der Farbstoffdichte, so daß keine Energietransfer-Messungen gemacht werden konnten.

Beispiel 8

Messung der Grenzempfindlichkeit des Förster-Energietransfersystems

Ein nach Beispiel 4 hergestelltes Schichtelement (Donorfarbstoff) wurde zusätzlich zunächst mit zwei Lagen des Polymers (V), dann mit einer Lage des Polymers (V), dem eine definierte Menge eines amphiphilen Akzeptorfarbstoffes zugemischt ist, belegt. An diesem Schichtelement wurde die Fluoreszenz gemessen. Durch Variation der Akzeptorfarbstoffmenge wurde diejenige Grenzkonzentration festgestellt, bei der die Fluoreszenz dieser Substanz gerade noch detektierbar war. Die folgende Tabelle stellt diese Werte für verschiedene Systeme dar:

| Donorfarbstoff | Akzeptorfarbstoff | Grenzkonzentration $[10^{-15}$ mol/mm$^2]$ |
|---|---|---|
| nach 4a | ($C_{18}$-Rhodamin) | 3 |
| nach 4b | | 0,3 |
| nach 4c | | 0,3 |
| nach 4c | ($C_{18}$-Aminofluorescein) | 3 |

Beispiel 9

Spezifische Bindung eines Mannosids an Concanavalin A

Analog zu Beispiel 4 wurde durch Übertragung eines gemischten Monofilms, bestehend aus der Verbindung der Formel (IIa) und Succinimidyl-stearat im Gewichtsverhältnis von 95:5, ein Schichtelement hergestellt. Auf dieses wurde anschließend eine Lösung von unmarkiertem Concanavalin A (1 mg/ml, gelöst in 0,01 mol/l Phosphatpuffer pH 6,8 mit 1 mmol/l $CaCl_2$, 1 mmol/l $MnCl_2$ und 0,01 % Triton X-100) eine Stunde lang bei Raumtemperatur einwirken gelassen (vergl. Beispiel 6). Das Element wurde mit 0,5 ml des gleichen Puffers gewaschen. Anschließend wurden 50 µl einer Lösung (0,1 mg/ml, gelöst im obigen Puffer) des TRITC-Mannosids (IX) aufgetragen und das Schichtelement erneut abgedeckt. In einem Kontrollversuch wurde anstelle von Con A die gleiche Menge Rinderserumalbumin verwendet.

Ein Vergleich der Fluoreszenzspektren beider Schichtelemente zeigt, daß bei Verwendung von Con A die Rhodamin-Emission bei 580 nm (aus IX) etwa fünffach stärker ist als die des Cumarins bei 495 nm (aus IIa). Bei Verwendung von Rinderserumalbumin ist nahezu keine (weniger als 1/20) Rhodamin-Emission im Vergleich zu der jetzt starken Cumarin-Emission zu beobachten.

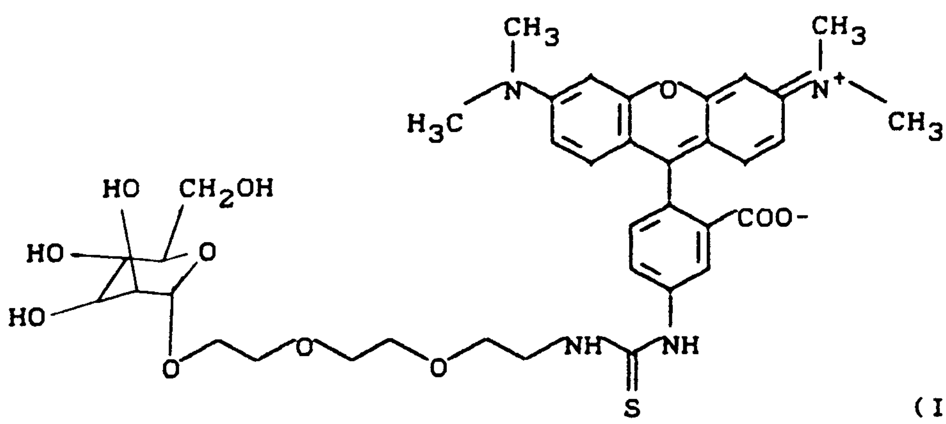

(IX)

## Patentansprüche

**1.** Optischer Biosensor auf der Basis des Fluoreszenz-Energietransfers, bestehend aus

a) einem festen Träger,

b) einer auf a) angebrachten ein- oder mehrlagigen Langmuir-Blodgett(LB)-Schicht,

c) mindestens einem Fluoreszenzfarbstoff $F_1$, der in mindestens einer der oberen 4 Lagen der LB-Schicht angeordnet ist,

d) einem zur spezifischen Wechselwirkung befähigten Molekül (Rezeptor), das in oder an der obersten Lage der LB-Schicht gebunden oder angeordnet ist, und

e) einem mobilen Fluoreszenzfarbstoff $F_2$, dessen Anregungsbande für einen Energietransfer ausreichend mit der Emissionsbande von $F_1$ überlappt und der

e1) kovalent an einen Liganden gebunden ist, der an den Rezeptor bindungsfähig ist oder der

e2) kovalent an einen anderen Rezeptor gebunden ist, der an den Komplex aus erstem Rezeptor und Ligand bindungsfähig ist,

wobei der Ligand bzw. der Ligand und der zweite Rezeptor zunächst nicht an die LB-Schicht gebunden sind.

**2.** Biosensor nach Anspruch 1, dadurch gekennzeichnet, das der verwendete feste Träger aus Glas, Quarzglas, Li-niobat, Zinkselenid, Porzellan, Halbleitermaterialien, wie Germanium, GaAs oder Silizium, einem Metall, einem Kunststoff oder einem metallisierten Kunststoff besteht, wobei der feste Träger oberflächenmodifiziert sein kann.

**3.** Biosensor nach Anspruch 1, dadurch gekennzeichnet, das die Matrix der LB-Schicht ein Polymer ist.

**4.** Biosensor nach Anspruch 1, dadurch gekennzeichnet, das $F_1$ kovalent an die Matrix gebunden ist.

**5.** Biosensor nach Anspruch 1, dadurch gekennzeichnet, das der Farbstoff $F_1$ mit einer amphiphilen Matrix kogespreitet ist, bevorzugt das ein Scheibe-Aggregate (J-Aggregate) bildender Farbstoff $F_1$ kogespreitet ist.

**6.** Biosensor nach Anspruch 1, dadurch gekennzeichnet, das der Farbstoff $F_1$ in mindestens einer der beiden oberen Lagen der LB-Schicht angeordnet ist.

**7.** Biosensor nach Anspruch 1, dadurch gekennzeichnet, das das Rezeptormolekül kovalent, bevorzugt über ein Spacermolekül, an die oberste LB-Schicht angebunden ist.

**8.** Biosensor nach Anspruch 1, dadurch gekennzeichnet, daß das Rezeptormolekül über einen hydrophoben Membrananker an die oberste Schicht angeordnet ist.

**9.** Biosensor nach Anspruch 1, dadurch gekennzeichnet, das an die Bindungsstellen der Rezeptormoleküle ein fluoreszenzfarbstoffmarkierter oder selbstfluoreszierender Ligand bindet oder daß ein nicht fluoreszenzmarkierter Analyt kompetitiv einen zunächst an die Bindungstellen der Rezeptormoleküle gebundenen und fluoreszenzmarkierten Liganden verdrängt.

**10.** Biosensor nach Anspruch 1, dadurch gekennzeichnet, das zu der Analytenlösung ein zweites, mit dem Fluoreszenzfarbstoff $F_2$ markiertes Rezeptormolekül zugesetzt wird, das entweder spezifisch an den Komplex aus Rezeptor und Analyt oder an einen anderen Molekülbereich des Analyten als der Rezeptor in der Art eines Sandwich-Assay bindet.

## Claims

**1.** Optical biosensor based on fluorescence energy transfer, consisting of

a) a solid support,

b) a single-layer or multilayer Langmuir-Blodgett (LB) film attached to a),

c) at least one fluorescent dye $F_1$ which is located in at least one of the top 4 layers of the LB film,

d) a molecule (receptor) which is capable of specific interaction and which is bound or located in or on the topmost layer of the LB film, and

e) a mobile fluorescent dye $F_2$ whose excitation band overlaps, sufficiently for an energy transfer, with the emission band of $F_1$ and which

e1) is covalently bonded to a ligand which is able to bind to the receptor, or which

e2) is covalently bonded to another receptor which is able to bind to the complex composed of the first receptor and ligand,

where the ligand or the ligand and the second receptor are initially not bound to the LB film.

**2.** Biosensor according to Claim 1, characterized in that the solid support used is composed of glass, quartz glass, Li niobate, zinc selenide, porcelain, semiconductor materials such as germanium, GaAs or silicon, a metal, a plastic or a metallized plastic, it being possible for the solid support to be surface-modified.

**3.** Biosensor according to Claim 1, characterized in that the matrix of the LB film is a polymer.

**4.** Biosensor according to Claim 1, characterized in that $F_1$ is covalently bonded to the matrix.

**5.** Biosensor according to Claim 1, characterized in that the dye $F_1$ is spread together with an amphiphilic matrix, preferably in that the spreading together is with a dye $F_1$ which forms disc aggregates (J aggregates).

**6.** Biosensor according to Claim 1, characterized in that the dye $F_1$ is located in at least one of the two upper layers of the LB film.

**7.** Biosensor according to Claim 1, characterized in that the receptor molecule is attached covalently, preferably via a spacer molecule, to the topmost LB film.

**8.** Biosensor according to Claim 1, characterized in that the receptor molecule is located onto the topmost film via a hydrophobic membrane anchor.

**9.** Biosensor according to Claim 1, characterized in that a ligand which is labelled with fluorescent dye or is self-fluorescent binds to the binding sites of the receptor molecules, or in that an analyte which is not fluorescent-labelled competitively displaces a fluorescent-labelled ligand which is initially bound to the binding sites of the receptor molecules.

**10.** Biosensor according to Claim 1, characterized in that a second receptor molecule which is labelled with the fluorescent dye $F_2$ and binds either specifically to the complex of receptor and analyte or to a different molecular region of the analyte than the receptor in the manner of a sandwich assay is added to the analyte solution.

## Revendications

**1.** Biocapteur optique basé sur le transfert d'énergie de fluorescence, constitué

a) d'un support solide,

b) d'une couche de Langmuir-Blodgett (LB) à une ou plusieurs strates disposée sur a),

c) d'au moins un colorant fluorescent $F_1$ qui est disposé dans l'une au moins des quatre strates supérieures de la couche LB,

d) d'une molécule (récepteur) susceptible d'interaction spécifique, qui est liée dans la strate supérieure de la couche LB ou disposée dans cette strate et,

e) d'un colorant fluorescent $F_2$ mobile dont la bande d'excitation pour un transfert d'énergie et la bande d'émission de $F_1$ se recouvrent suffisamment, et qui

e1) est lié par covalence à un ligand qui est capable de se lier au récepteur, ou qui

e2) est lié par covalence à un autre récepteur qui est susceptible de liaison au complexe formé du premier récepteur et du ligand,

le ligand ou le ligand et le second récepteur n'étant tout d'abord pas liés à la couché LB.

**2.** Biocapteur suivant la revendication 1, caractérisé en ce que le support solide utilisé est constitué de verre, de verre au quartz, de niobiate de lithium, de séléniure de zinc, de porcelaine, de matières semiconductrices telles que germanium, GaAs ou silicium, d'un métal, d'une matière plastique ou d'une

matière plastique métallisée, le support solide pouvant être modifié en surface.

3. Biocapteur suivant la revendication 1, caractérisé en ce que la matrice de la couche LB est un polymère.

4. Biocapteur suivant la revendication 1, caractérisé en ce que $F_1$ est en liaison de covalence avec la matrice.

5. Biocapteur suivant la revendication 1, caractérisé en ce que le colorant $F_1$ est en co-extension avec une matrice amphiphile, de préférence en ce qu'un colorant $F_1$ formant des agrégats J est en co-extension.

6. Biocapteur suivant la revendication 1, caractérisé en ce que le colorant $F_1$ est disposé dans l'une au moins des deux strates supérieures de la couche LB.

7. Biocapteur suivant la revendication 1, caractérisé en ce que la molécule de récepteur est liée par covalence, de préférence par l'intermédiaire d'une molécule intercalaire, à la couche LB la plus haute.

8. Biocapteur suivant la revendication 1, caractérisé en ce que la molécule de récepteur est disposée par l'intermédiaire d'un élément d'ancrage à membrane hydrophobe sur la couche la plus haute.

9. Biocapteur suivant la revendication 1, caractérisé en ce qu'un ligand marqué par un colorant fluorescent ou un ligand auto-fluorescent est lié aux sites de liaison des molécules de récepteur ou en ce qu'un analyte non marqué par la fluorescence déplace par compétition un ligand tout d'abord lié aux sites de liaison de la molécule de récepteur et marqué par la fluorescence.

10. Biocapteur suivant la revendication 1, caractérisé en ce qu'on ajoute à la solution d'analyte une seconde molécule de récepteur marqué avec le colorant fluorescent $F_2$, qui se lie spécifiquement au complexe formé du récepteur et de l'analyte ou à une région de la molécule de l'analyte autre que le récepteur, à la manière d'une épreuve sandwich.